# EUROPÄISCHE PATENTSCHRIFT

(11) **EP 1 384 494 B1**
(45) Veröffentlichungstag und Bekanntmachung des Hinweises auf die Patenterteilung: **11.05.2005**
(21) Anmeldenummer: 03024993.2
(22) Anmeldetag: 06.06.2001
(51) Int. Cl.: A61N 5/10, A61B 6/04

(54) **Patientenpositionierungssystem für die Radiotherapie**
Patient positioning system for radiotherapy
Système de positionnement d'un patient pour la radiothérapie

(30) Priorität: 05.03.2001 EP 01104553
(43) Veröffentlichungstag der Anmeldung: 28.01.2004
(62) Teilanmeldung aus: 01112942.6
(73) Patentinhaber: BrainLAB AG, 85551 Kirchheim/Heimstetten (DE)
(72) Erfinder: Erbel, Stephan, 81677 München (DE); Fröhlich, Stephan, 85609 Aschheim (DE)
(74) Vertreter: Schwabe - Sandmair - Marx

(56) Entgegenhaltungen:
- EP-A- 0 047 958
- EP-A- 0 370 567
- EP-A- 0 562 585
- WO-A-99/27839
- DE-A- 2 260 140
- DE-A- 4 341 779
- DE-A- 19 612 091
- DE-A- 19 920 008
- US-A- 3 818 516
- US-A- 4 905 267
- US-A- 5 475 884
- US-A- 5 851 182

## Beschreibung

Die vorliegende Erfindung betrifft eine Patientenpositionierungsvorrichtung für die Radiotherapie bzw. für die Bestrahlungstherapie oder Radiochirurgiegemäß dem Oberbegriff des Patentanspruchs 1. Eine solche Vorrichtung ist für den Bereich angiographischer Untersuchungen aus der DE 199 20 008 A1 bekannt.

Aus der DE 43 41 779 A1, der DE 196 12 091 A1 und der DE-OS 2 260 140 sind Vorrichtungen zur Patientenpositionierung mit einer Patientenliege bekannt, auf der ein Patient gelagert wird, die eine Verstelleinrichtung aufweisen, mit der die Patientenliege um mindestens zwei Achsen gedreht werden kann und die zwischen der Patientenliege und einer Stützeinrichtung für die Patientenliege angeordnet ist. Die US 3, 818,516 zeigt ein Krankenhausbett das in der Höhe mittels Kniehebeln verstellbar ist.

In den meisten Fällen ist bei der Bilderfassung und bei der Bestrahlung der Rotationswinkel um die Körperlängsachse und die horizontale Querachse hierzu nicht identisch. Deshalb ist der Weg, den der Strahl durchschreitet, bis er zum Behandlungsziel kommt meist nicht identisch mit dem Weg, der bei der Bestrahlungsplanung im Sinne einer optimalen Behandlung festgelegt wurde. Stereotaktische Radiochirurgie setzt die Möglichkeit voraus, ein bestimmtes Zielgebiet im Körper des Patienten aus einer bestimmten Richtung bestrahlen zu können. Durch die begrenzte Steifigkeit der Tische von Linearbeschleunigern und ähnlichen Radiochirurgie- und Radiotherapiesystemen kommt es jedoch oft dazu, dass das zu behandelnde Körperteil oder der gesamte Patient nach der Fixierung auf dem Behandlungsgerät eine Winkelverdrehung erfährt, die entsprechen korrigiert werden muss.

Um diese Korrektur durchzuführen, werden beim Stand der Technik andererseits auch z.B. Systeme verwendet, bei denen der Kopf des Patienten innerhalb eines bestimmten Winkelbereiches um die Quer- und Längsachse gedreht werden kann. Ein solches Patiententisch- bzw. Patientenliegesystem mit einer Verstelleinrichtung gemäß dem Stand der Technik ist in schematischer Darstellung in Figur 10 gezeigt. Die Verstelleinrichtung C wird auf der Behandlungsliege B des Therapiegerätes befestigt und nimmt den Kopf mittels eines Masken- oder Schraubringsystems D auf. Um die nötige Steifigkeit zu erreichen, sind diese Verstellmechanismen schwer und relativ groß. Um die Kräfte und Momente aus ihrem eigenen Gewicht und dem Gewicht des Patientenkopfes aufzunehmen und auf die Behandlungsliege übertragen zu können, ist es dabei notwendig, relativ solide und schwere Aufnahmevorrichtungen am Patiententisch vorzusehen. Diese Aufnahmevorrichtungen sind jedoch insofern problematisch, als sie nicht so strahlungsdurchlässig sein können wie eine einfache, durchgehende (Karbon-)Liege. Ein zusätzliches Problem ist die Größe der Vorrichtungen, die das Kollisionsrisiko zwischen Liege und dem bewegten Teil des Bestrahlungsgerätes deutlich erhöht, und den Transport der Liege wesentlich erschwert. Außerdem kann die Winkelverstellung nicht für extrakranielle Bestrahlungen genutzt werden, und gerade dieser Mangel ist durch die in letzter Zeit erzielten Fortschritte auf dem Gebiet der extrakraniellen Radiotherapie ein wesentlicher Nachteil.

Die erfindungsgemäße Vorrichtung wird durch den Patentanspruch 1 definiert. Die Unteransprüche definieren bevorzugte Ausführungsformen der Erfindung.

Eine Kombination mit einem Erfassungssystem (Trackingsystem) ist möglich.

Die Verstelleinrichtung kann ein unabhängiges Drehen der Patientenliege um mindestens eine Achse quer und eine Achse längs zur Liege ermöglichen. Sie besteht aus einer Grund- und einer Deckplatte, die über eine Vierpunktlagerung miteinander beweglich verbunden sind. Hierbei ist es möglich, die Verstelleinrichtung so auszugestalten, dass sie mindestens zwei Auflager aufweist, die als höhenverstellbare, pneumatische, hydraulische, piezoelektrische oder elektromechanische Auflager, direkt oder mittels eines Hebelsystems verstellbar, ausgebildet sind.

Die Verstelleinrichtung weist bevorzugt eine vordere, kopfseitige Auflagerung und eine hinter, fußseitige Auflagerung auf, wobei mindestens eine der Auflagerungen als Kugelgelenk- oder Kardanlage ausgebildet ist. Die vordere oder hintere Auflagerung kann dabei Kniehebel mit beidseitigen Kugelgelenken aufweisen.

Bei einer Ausgestaltung der erfindungsgemäßen Vorrichtung umfasst die Lagerung der Deckplatte auf der Grundplatte ein Wellen-Nut-Gelenk mit zwei translatorischen und zwei rotatorischen Freiheitsgraden, welches ein seitliches Verschieben der Deckplatte gegenüber der Grundplatte verhindert. Einerseits kann die Vorrichtung Verstellmittel aufweisen, welche die Rotation der Liege um ihre Längsachse durch gegenläufiges Verstellen von zwei kopfoder fußseitigen Auflagern bewirken, andererseits können die Verstellmittel auch die Rotation der Liege um ihre Querachse durch gleichläufiges Verstellen eines oder zweier kopf- oder fußseitiger Auflager bewirken. Natürlich kann beides realisiert werden. Die Verstellmittel können ebenfalls die Rotation der Liege um ihre Querachse durch gleichzeitiges Verstellen mindestens eines kopfseitigen und mindestens eines fußseitigen Auflagers bewirken.

Insbesondere bei der Verwendung von Kniehebeln muss die Verstell-Geschwindigkeit beachtet werden. Deshalb weist die Vorrichtung bei einer Ausführungsform Verstellmittel auf, welche die Rotation der Liege um ihre Querachse durch gezieltes Einstellen der Verstell-Geschwindigkeit mindestens eines kopfseitigen und mindestens eines fußseitigen Auflagers bewirken, wobei die Lage der scheinbaren Drehachse in eine gewollte Position gebracht werden kann. Ferner kann sie Verstellmittel aufweisen, welche die Rotation der Liege um ihre Längsachse durch gezieltes Einstellen der Verstell-Geschwindigkeit mindestens zweier Auflager bewirkt, wodurch die Lage der scheinbaren Drehachse in eine gewollte Position gebracht werden kann.

Es wird ferner eine Vorrichtung zur Patientenpositionierung im Rahmen der Behandlungsplanung mit Bilderfassung und/oder der Durchführung einer Bestrahlungsplanung offenbart, mit einer Patientenliege, auf der ein Patient gelagert wird, insbesondere eine Vorrichtung, wie sie oben beschrieben wurde, wobei diese Vorrichtung eine Transportvorrichtung, insbesondere einen Transportwagen zum Transportieren der Liege und des darauf liegenden Patienten umfasst. Eine solche Transportvorrichtung kann in optimaler Weise die schon vorher beschriebenen vorteilhaften Ausführungsformen der Patientenlagerung ergänzen, und bei einer bevorzugten Ausgestaltung sind dabei zur Verbindung von Patientenliege und Transportwagen zwei Einklinkmechanismen vorgesehen, zum Einhängen der Patientenliegen an einem Tisch eines Behandlungssystems und an einer Couch eines bildgebenden Planungssystems. Das Ein- und Aushängen der Liege beim Tisch des Bestrahlungsgerätes oder bei der Couch des Planungssystems kann einerseits über die Höhenverstellung des Tisches bzw. der Couch erfolgen; andererseits besteht auch die Möglichkeit, dies über die Höhenverstellung der Transportvorrichtung zu realisieren.

Die Erfindung wird im Weiteren anhand bevorzugter Ausführungsformen näher erläutert. In den beiliegenden Zeichnungen zeigen:
- Figur 1: ein Patiententisch-Patientenliege-System mit Verstelleinrichtung gemäß der vorliegenden Erfindung in einer Schrägansicht bei einem Bilderfassungssystem und in einer Seitenansicht, wie es zur Bestrahlung verwendet wird;
- Figur 2: eine Verstelleinrichtung gemäß der Erfindung in oberer und seitlicher Schnittansicht;
- Figur 3: ein hinteres Lager der Verstelleinrichtung in seitlicher, vergrößerter Ansicht;
- Figur 4: eine Frontalansicht einer Ausführungsvariante der Verstelleinrichtung mit zwei Kniehebeln und einem zusätzlichen Führungsgelenk;
- Figur 5: Schnitt-Seitenansichten, die eine Verstellmöglichkeit für die Kniehebel der Verstelleinrichtung illustrieren;
- Figur 6: eine Frontalansicht der Verstelleinrichtung bei gegenläufigem Verstellen zweier Kniehebel;
- Figur 7: eine Ausführungsvariante einer selbsttragenden Karbonliege auf der Couch eines konventionellen Linearbeschleunigers;
- Figur 8: eine Ausführungsform eines Transportsystems für eine Patientenliege;
- Figur 9: eine Ausführungsform eines Mechanismus zum Ein- und Ausklinken der Liege auf der Verstelleinrichtung oder direkt auf der Couch; und
- Figur 10: ein Patiententisch-Patientenliege-System mit Verstelleinrichtung gemäß dem Stand der Technik in schematischer Darstellung.

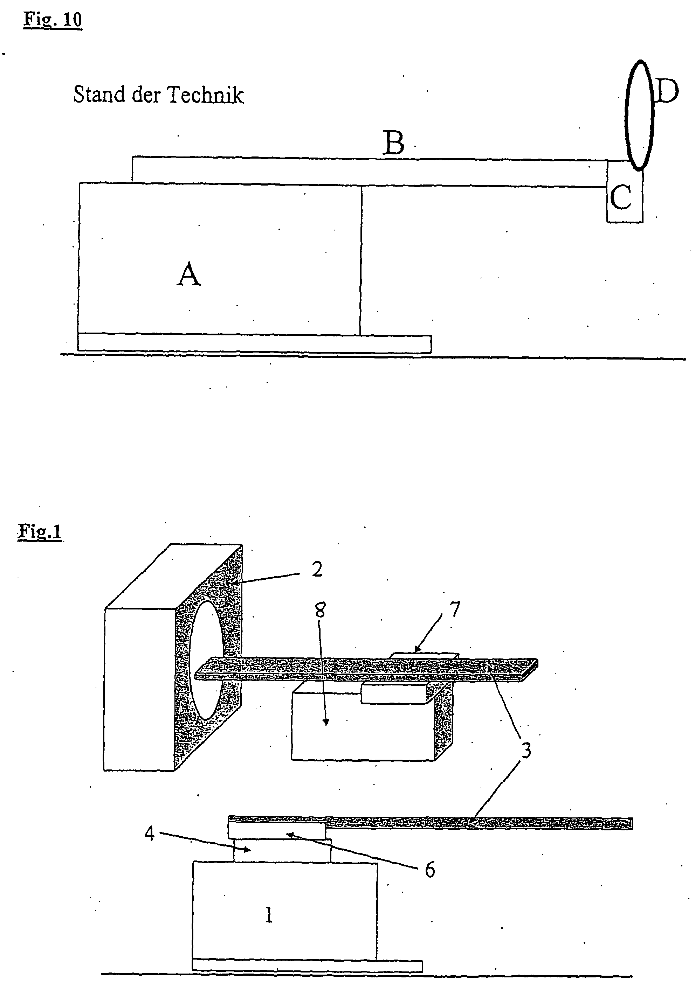

Die Figur 1 zeigt ein Patiententisch-Patientenliege-System mit Verstelleinrichtung gemäß der vorliegenden Erfindung in einer Schrägansicht bei einem Bilderfassungssystem (Planung) und einer Seitenansicht, wie es beispielsweise bei einer Bestrahlungstherapie unter einem LINAC (LINear ACcellerator) verwendet wird. Dargestellt sind in der oberen Schrägansicht (beim Planungsschritt): ein Planungssystem bestehend aus einer Patientencouch 8 und einem bildgebenden Gerät 2, beispielsweise einem CT-Gerät; und ein schematisch dargestellter Klinkmechanismus 7, mit dem eine Patientenliege 3 an der Couch 8 befestigt werden kann. In der unteren Darstellung ist die Liege 3 zur Bestrahlung auf einem Patiententisch 1 aufgebracht und eine Verstelleinrichtung 6 ist zwischen einem weiteren Klinkmechanismus 4 am Patiententisch 1 und der Patientenliege 3 eingesetzt.

Anstatt der bisher üblichen Anordnungsreihenfolge der Verbindungsstruktur: Tisch (A)-Liege (B)-Verstellmechanismus (C) (siehe Figur 10) wird die Verbindungsstruktur: Tisch (1, 8) - Verstellmechanismus (6)- Liege (3) implementiert. Dadurch lässt sich demnach die Winkelkorrektur auch für Behandlungen außerhalb des Kopfbereiches nutzen. Der zweite Vorteil dieser Vorgehensweise ist, dass die Liege 3, auf der der Patient liegt, auch im Halsbereich durchgehend aus gut strahlungsdurchlässigem Karbonmaterial gestaltet werden kann; Verstärkungen, Auflager- und Verschraubungspunkte auf der Patientenliege 3 entfallen vollständig.

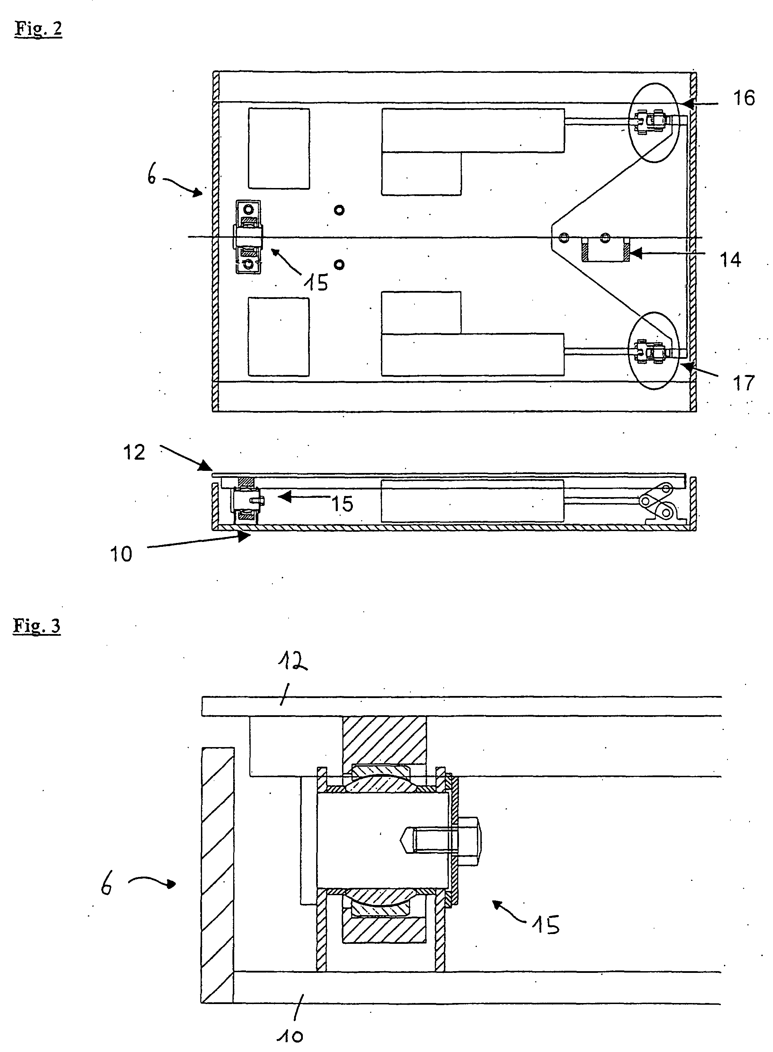

Da das hier dargestellte System auch dazu gedacht ist, bereits existierende Bestrahlungs-Systeme aufzurüsten, ist es besonders wichtig, dass die Verstelleinrichtung 6 in einen stark eingeschränkten Bauraum integriert werden kann. Diese Anforderung ist jedoch mit konventionellen Systemen mit zwei Drehachsen nicht realisierbar. Das erfundene System nutzt deshalb eine spezielle Aufhängung in der Verstelleinrichtung, wobei eine bevorzugte Ausführung im Folgenden anhand der Figur 2 beschrieben wird.

Die Figur 2 zeigt eine Verstelleinrichtung 6 gemäß der Erfindung in oberer und seitlicher Schnittansicht. Sie weist eine Grundplatte 10 und eine Deckplatte 12, auf. Die Verbindung zwischen der Grundplatte 10 auf dem Tisch des Behandlungsgerätes und der Deckplatte 12 auf der die (Karbon-) Liege 3 befestigt wird, ist über eine Dreipunktlagerung 15, 16, 17, die durch ein zusätzliches Seitenführungsgelenk 14 ergänzt wird, realisiert. Das hintere Lager 15 (etwa auf Höhe der Füße des Patienten) ist ein Kugel-Gelenk-Lager mit drei rotatorischen und keinem translatorischen Freiheitsgrad. Es ist in Figur 3 nochmals vergrößert gezeigt. Alternativ kann dieses Lager auch als Kardangelenk ausgeführt werden.

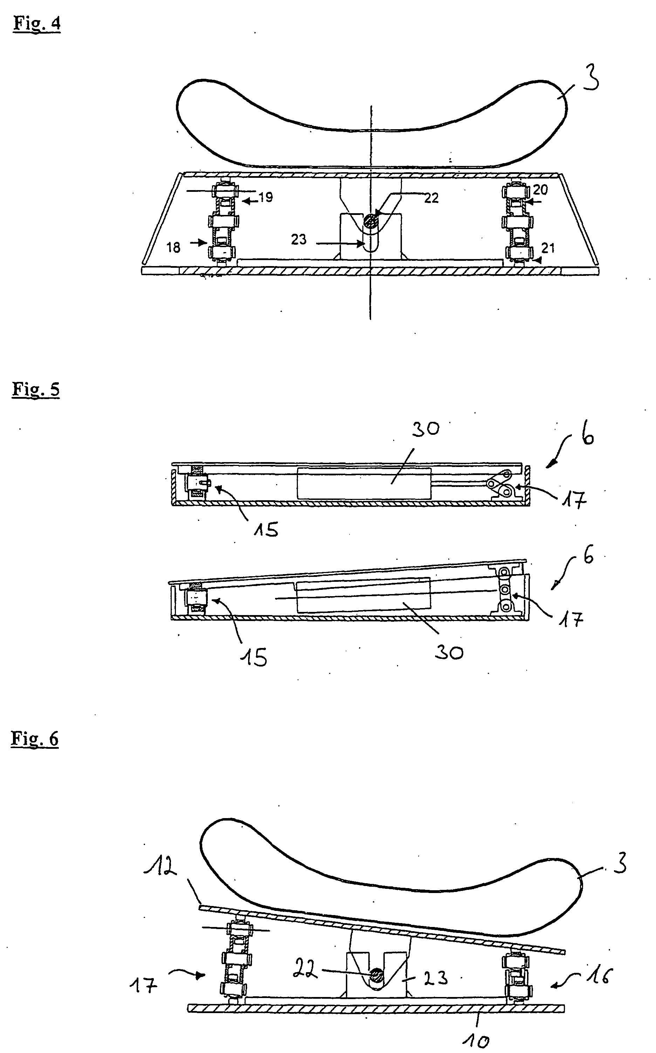

Die vorderen zwei Auflager 16, 17 bestehen aus Kniehebeln, die an der Grund- und Deckplatte 10, 12 jeweils mit Kugelgelenken 18, 19, 20, 21 fixiert sind, die in der Frontalansicht der Figur 4 am besten zu sehen sind.

Das Seitenführungsgelenk 14 befindet sich in der Nähe der Mittelgeraden zwischen beiden Kniehebeln, und weist zwei rotatorische und zwei translatorische Freiheitsgrade auf. Eine bevorzugte Ausführung dieses Gelenks ist eine Welle 22, die in einem Spalt 23 geführt wird.

In Figur 5 sind Schnitt-Seitenansichten dargestellt, die eine Verstellmöglichkeit für die Kniehebel 16, 17 der Verstelleinrichtung 6 illustrieren, und die Figur 6 zeigt eine Frontalansicht der Verstelleinrichtung 6 bei gegenläufigem Verstellen zweier Kniehebel 16, 17. Die zwei Kniehebel 16, 17 können über Elektrozylinder (30) (je ein Spindel-Mutter-System), hydraulische Zylinder oder pneumatische Zylinder angesteuert werden.

Werden die beiden Kniehebel 16, 17 in die gleiche Richtung gespreizt, so lässt sich die Liege 3 um eine Achse quer zum Patienten drehen, werden die Kniehebel 16, 17 in unterschiedliche Richtungen gespreizt, so lässt sich ein Drehen um eine Achse die in etwa parallel zur Längsachse des Patienten ist realisieren.

Um ein Drehen um die Hauptachsen (Achse durch das hintere Kugelgelenk 15, senkrecht zur Symmetrieebene des Patienten, und Achse parallel zur Symmetrieebene des Patienten) zu realisieren, ist es notwendig die effektive Höhe der Kniehebel 16, 17 mit gleicher beziehungsweise gegenläufiger Geschwindigkeit zu verändern. Eine solche gegenläufige Verstellung führt beispielsweise zu einem Zustand, wie er in der Figur 6 dargestellt ist. Da die Kniehebel 16, 17 eine vom Einknickwinkel abhängige Weg- und Kraftübersetzung aufweisen, ist es dazu nötig die Kniehebel mit Geschwindigkeiten unterschiedlichen Betrags, die in Abhängigkeit vom Knickwinkel beider Kniehebel errechnet werden, anzusteuern. Ist dies realisiert, so ist es möglich die Liege 3 um die Längsachse zu drehen, ohne dass dadurch der Winkel um die Querachse oder die Höhe der Liege 3 verändert wird.

Alternativ kann das Kugelgelenk 15 auch durch einen dritten Kniehebel mit einseitigem Kugelgelenk und einem einfachen Drehlager auf der anderen Seite dieses Kniehebels ausgeführt werden (nicht dargestellt). Bei einer solchen Ausführungsform befindet sich anstelle des Kugelgelenklagers der dritte Kniehebel, der ebenfalls über Kugelgelenklager an der Grund- und Deckplatte befestigt wird. Mit Hilfe dieses dritten Hebels ist es zusätzlich möglich, den Patienten um eine virtuelle Querachse zu drehen, deren Lage durch das Verhältnis der effektiven Geschwindigkeiten des einzelnen Kniehebels bezogen auf die effektive Geschwindigkeit der beiden Kniehebel auf der anderen Seite eingestellt werden kann. Auf diese Weise ist es möglich den auf der Liege liegenden Patienten um einen bestimmten Punkt zu drehen, vorzugsweise um den Punkt der behandelt werden soll. Im Falle eines Linearbeschleunigers ist dies das Isozentrum.

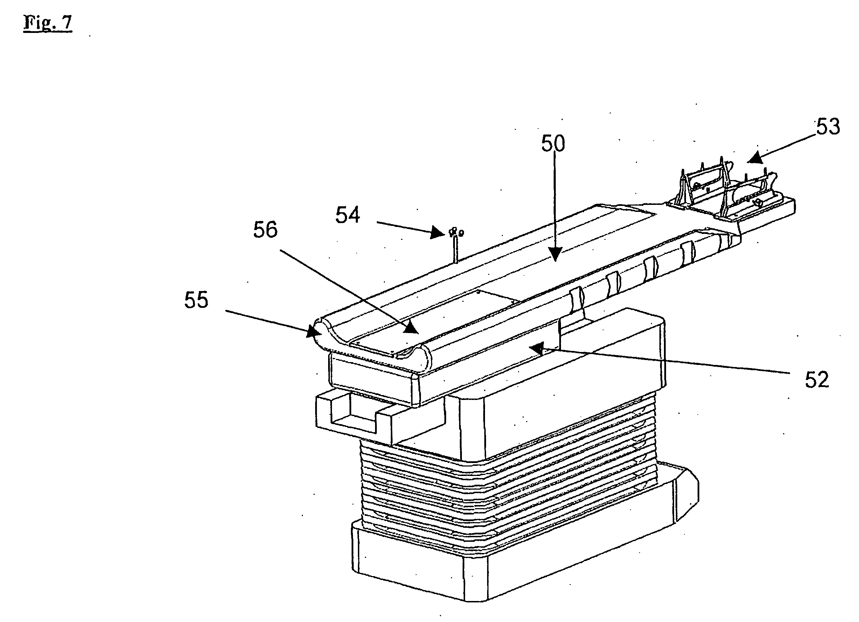

Die Figur 7 zeigt eine mögliche Ausführungsform einer Patientenliege 50 auf der Verstelleinrichtung 6. Ein Kopfhalter 53 ist gezeigt, und dieser kann nicht verdreht und deswegen aus sehr dünnem und dadurch strahlungsdurchlässigem Karbon realisiert werden. Bei 55 kann eine Endplatte aus Metall vorgesehen werden, die dazu dienen kann, die Liege 50 an einer Verstelleinrichtung 52 zu befestigen, oder auch dazu genutzt werden kann, Fußhalter oder ähnliche Zusatzgeräte mit der Liege 50 zu verbinden. Auf einem Referenzstern 54 sind IR-reflektierende Marker angebracht, die als Referenzmarkierungen für auf dem Patienten befindliche Marker genutzt werden können. Ein in die Liege integrierte Rahmen 56 ist wesentlich kürzer als die eigentliche Liege wodurch er nicht in Bereiche ragt, die strahlungsdurchlässig sein sollen.

Damit die Position des Patienten auf der Liege 50 während der Behandlung noch die gleiche ist wie die während der Aufnahme des Planungsdatensatzes darf sich der Patient zwischen diesen Zeitpunkten nicht bewegen. Die Liege wird deshalb vorzugsweise mit einem Patientenfixierungssystem kombiniert. Dazu sind unter anderen geeignet:
- Vakuumfolien mit entsprechenden Unterdruckpumpen
- Elastische Bänder oder Folien, die über den Patienten gespannt werden
- Speziell an die Form des Patienten angepasste Vakuumkissen
- Speziell an die Form des Patienten angepasste thermoplastische Körpermasken
- Formkissen oder -blöcke, die an die Form des Patienten angepasst werden

Der Stern 54 mit den Referenzmarkern ist fest mit der Liege 50 verbunden. Durch die Atmung des Patienten ändert sich der Abstand zwischen den Referenzmarkern und eventuellen Markern auf dem Thorax des Patienten. Dieser Abstand ist dazu geeignet die Atmung des Patienten zu beschreiben. Die Korrelation zwischen diesem Abstandswert und der Position interner Organe des Patienten bleibt erhalten, wenn die Liege 50 transportiert wird, da sowohl die Referenzmarker als auch der Patient fest mit der Liege verbunden sind. Ein (nicht dargestelltes) Trackingsystem am Planungsort (zum Beispiel CT) und am Behandlungsort (LINAC) unterstützt zum Beispiel eine atmungsgesteuerte Planung und Bestrahlung (getriggerte CT-Aufnahmen und getriggerte Bestrahlung), wobei dies erst möglich wird, weil die oben genannte Korrelation erhalten bleibt. Ein gewisser Toleranzbereich ist dabei einzuhalten. Die automatische Positionierung der Liege 50 am LINAC (computergestützt über das Trackingsystem und die Marker am Stern 54 an der Liege) erfolgt ebenfalls erst bei Erreichen des definierten Verhältnisses. Als Erweiterung ist es denkbar, an mehreren definierten Patienten-Fix-Marker Zuständen verschiedene CT-Sätze des gleichen Gebiets aufzunehmen, um mehrere "Anfangspunkte" schon beim CT bereitzustellen. Ein Arzt kann dann die Bewegung des Zielpunkts analysieren und ein "Beam-On-Range" bei der Bestrahlung (erlaubte Patienten-Fix-Marker-Beziehung für "Beam-On") kann individuell definiert werden. Wenn hier von CT-Planung die Rede ist, so soll dieses Verfahren nur als ein Beispiel eines bildgebenden Verfahrens stehen. Grundsätzlich ist die Verwendung anderer Verfahren möglich, zum Beispiel MR, SPECT, PET.

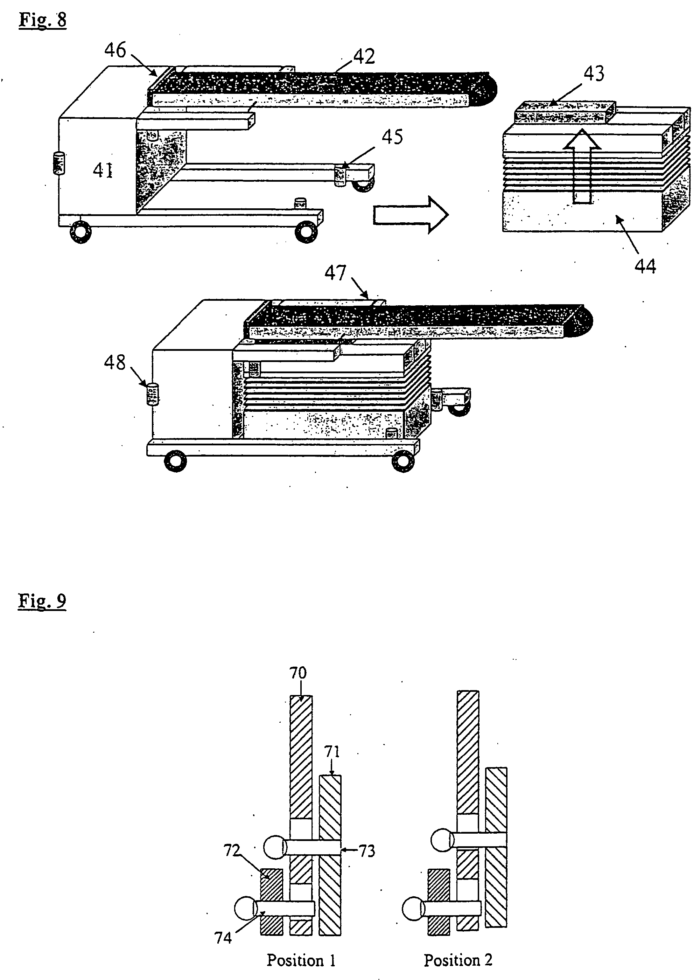

Um die Vorteile einer transportablen Patientenliege gut nutzen zu können, ist es vorteilhaft eine Vorrichtung zu haben, die es erlaubt, die Liege samt Patient vom bildgebenden System zum Behandlungsgerät transportieren zu können. Die Figur 8 zeigt eine Ausführungsform eines Transportsystems für eine Patientenliege 42. Ein Transportwagen 41 hält die Liege 42 in in etwa horizontaler Lage. Wird der Wagen 41 über den Tisch 44 des Behandlungsgerätes oder des bildgebenden Systems gefahren, so kann die Liege 42 auf die Verstelleinrichtung 43 übergeben werden. Der Wagen 41 ist mit seitlichen Rollen 45 an der Innenseite der zwei unteren Träger bestückt, so dass der Wagen 41 sich selbsttätig gegenüber dem Tisch 44 zentriert. Der Wagen 41 ist mit seitlichen Rollen 48 am liegenabgewandten Ende bestückt, sodass es möglich ist mit dem Wagen 41 Türen aufzuschieben.

Die Figur 9 zeigt eine mögliche Ausführungsform des Mechanismus zum Ein- und Ausklinken der Liege auf der Verstelleinrichtung oder direkt auf der Couch. Der wesentliche Vorteil dieses Mechanismus liegt darin, dass keine nennenswerte Handkraft aufgebracht werden muss, da die notwendige Kraft vom Tisch aufgebracht wird. Sowohl die Tische von Linearbeschleunigern als auch die von MR- und CT-Systemen erlauben es die Höhe des Tisches zu verstellen. Eine fest mit der Patientenliege verbundene Endplatte 70 (Bezugszeichen 46 in Figur 8) wird durch das vom Gewicht des überhängenden Patienten stammende Drehmoment nach oben gezogen. Ein Bolzen 73, der die Endplatte 70 mit dem Verstellmechanismus oder der Couch 71 verbindet, ist somit konstant mit einer Querkraft belastet. Der Bolzen 73 ist vorzugsweise mit Hinterschnitt gestaltet (in Figur 9 nicht sichtbar), so dass er nicht herausgezogen werden kann, wenn er mit Kraft belastet ist. Ein zweiter Bolzen 74 wird durch die mit dem Transportmittel für die Liege verbundenen Platte 74 in ein Langloch in die Endplatte 70 geführt. Solange der Bolzen 73 belastet ist, ist der Bolzen 74 unbelastet, so dass dieser ohne nennenswerte Kraft umgesteckt werden kann. Die Position 1 zeigt den Fall, bei dem die Liege mit dem Verstellmechanismus verbunden ist (Bolzen 73 trägt die Kraft).

Wird jetzt das Transportsystem an die Liege herangefahren, so können durch ein Herauffahren des Tisches die Liege und die Verstelleinrichtung und damit die Platten 70 und 71 nach oben gefahren werden, bis der Bolzen 74 am unteren Ende des Langloches anschlägt und von da an den Kraftfluss übernimmt. Wird der Tisch noch einige Millimeter weiter nach oben gefahren, so wird der Bolzen 73 ganz entlastet und kann von Hand herausgezogen werden. In umgekehrter Reihenfolge erfolgt die Abtrennung der Liege durch den Klinkmechanismus.

## Patentansprüche

1. Vorrichtung zur Patientenpositionierung im Rahmen der Behandlungsplanung mit Bilderfassung und/oder der Durchführung einer Bestrahlungsbehandlung, mit einer Patientenliege (3, 42, 50), auf der ein Patient gelagert wird, und mit einer Verstelleinrichtung (6, 52), mit der die Patientenliege (3, 42, 50) um mindestens zwei Achsen gedreht werden kann und die zwischen der Patientenliege (3, 42, 50) und einer Stützeinrichtung für die Patientenliege (3, 42, 50), insbesondere einer Planungscouch (8) bzw. einem Behandlungstisch (1) angeordnet ist, wobei die Verstelleinrichtung (6, 52) aus einer Grund- und einer Deckplatte (10, 12) besteht, die über eine Vierpunktlagerung miteinander beweglich verbunden sind, **dadurch gekennzeichnet, dass** die Lagerung der Deckplatte (12) auf der Grundplatte (10) ein Gelenk (22, 23) mit zwei translatorischen und zwei rotatorischen Freiheitsgraden umfasst, welches ein seitliches Verschieben der Deckplatte (12) gegenüber der Grundplatte (10) verhindert.

2. Vorrichtung nach Anspruch 1, **dadurch gekennzeichnet, dass** die Verstelleinrichtung (6, 52) ein unabhängiges Drehen der Patientenliege (3, 42, 50) um mindestens eine Achse quer und/oder eine Achse längs zur Liege (3, 42, 50) ermöglicht.

3. Vorrichtung nach Anspruch 1 oder 2, **dadurch gekennzeichnet, dass** die Verstelleinrichtung (6, 52) mindestens zwei Auflager (16, 17) aufweist, die als höhenverstellbare, pneumatische, hydraulische, piezoelektrische oder elektromechanische Auflager, direkt oder mittels eines Hebelsystems verstellbar, ausgebildet sind.

4. Vorrichtung nach Anspruch 1 bis 3, **dadurch gekennzeichnet, dass** die Verstelleinrichtung (6, 52) eine vordere, kopfseitige Auflagerung und eine hintere, fußseitige Auflagerung aufweist, wobei mindestens eine der Auflagerungen als Kugelgelenk- oder Kardanlager ausgebildet ist.

5. Vorrichtung nach Anspruch 4, **dadurch gekennzeichnet, dass** die vordere oder die hintere Auflagerung Kniehebel mit beiseitigen Kugelgelenken aufweist.

6. Vorrichtung nach einem der Ansprüche 3 bis 5, **dadurch gekennzeichnet, dass** sie Verstellmittel aufweist, welche die Rotation der Liege (3, 42, 50) um ihre Längsachse durch gegenläufiges Verstellen von zwei kopf- oder fußseitigen Auflagern bewirken.

7. Vorrichtung nach einem der Ansprüche 3 bis 6, **dadurch gekennzeichnet, dass** sie Verstellmittel aufweist, welche die Rotation der Liege (3, 42, 50) um ihre Querachse durch gleichläufiges Verstellen eines oder zweier kopf- oder fußseitiger Auflager bewirken.

8. Vorrichtung nach einem der Ansprüche 3 bis 7, **dadurch gekennzeichnet, dass** sie Verstellmittel aufweist, welche die Rotation der Liege (3, 42, 50) um ihre Querachse durch gleichzeitiges Verstellen mindestens eines kopfseitigen und mindestens eines fußseitigen Auflagers bewirken.

9. Vorrichtung nach einem der Ansprüche 3 bis 8, **dadurch gekennzeichnet, dass** sie Verstellmittel aufweist, welche die Rotation der Liege (3, 42, 50) um ihre Querachse durch gezieltes Einstellen der Verstell-Geschwindigkeit mindestens eines kopfseitigen und mindestens eines fußseitigen Auflagers bewirken, wodurch die Lage der scheinbaren Drehachse in eine gewollte Position gebracht werden kann.

10. Vorrichtung nach einem der Ansprüche 3 bis 9, **dadurch gekennzeichnet, dass** sie Verstellmittel aufweist, welche die Rotation der Liege (3, 42, 50) um ihre Längsachse durch gezieltes Einstellen der Verstell-Geschwindigkeit mindestens zweier Auflager bewirkt, wodurch die Lage der scheinbaren Drehachse in eine gewollte Position gebracht werden kann.

## Claims

1. A device for positioning a patient within the framework of treatment planning using image detection and/or carrying out radiation treatment, comprising a patient bed (3, 42, 50) on which a patient is positioned, and comprising an adjusting device (6, 52) with which the patient bed (3, 42, 50) may be rotated about at least two axes and which is arranged between the patient bed (3, 42, 50) and a supporting device for the patient bed (3, 42, 50), in particular a planning couch (8) or a treatment table (1), wherein the adjusting device (6, 52) consists of a base plate and a cover plate (10, 12) which are movably linked together via a four-point bearing, **characterised in that** the bearing of the cover plate (12) on the base plate (10) includes a joint (22, 23) with two translatory and two rotatory degrees of freedom, which prevents the cover plate (12) from shifting laterally with respect to the base plate (10).

2. The device as set forth in claim 1, **characterised in that** the adjusting device (6, 52) enables the patient bed (3, 42, 50) to turn independently about at least one axis laterally and/or one axis longitudinally to the bed (3, 42, 50).

3. The device as set forth in claim 1 or 2, **characterised in that** the adjusting device (6, 52) comprises at least two supports (16, 17) which are constructed as height-adjustable, pneumatic, hydraulic, piezoelectric or electromechanical supports, adjustable directly or by means of a lever system.

4. The device as set forth in claims 1 to 3, **characterised in that** the adjusting device (6, 52) comprises a front, head-end support and a rear, foot-end support, wherein at least one of the supports is constructed as a ball joint or as a universal joint.

5. The device as set forth in claim 4, **characterised in that** the front or the rear support comprises toggle levers with ball joints on both sides.

6. The device as set forth in any one of claims 3 to 5, **characterised in that** it comprises adjusting means which cause the bed (3, 42, 50) to rotate about its longitudinal axis by counter-adjusting two head-end or foot-end supports.

7. The device as set forth in any one of claims 3 to 6, **characterised in that** it comprises adjusting means which cause the bed (3, 42, 50) to rotate about its lateral axis by synchronously adjusting one or two head-end or foot-end supports.

8. The device as set forth in any one of claims 3 to 7, **characterised in that** it comprises adjusting means which cause the bed (3, 42, 50) to rotate about its lateral axis by simultaneously adjusting at least one head-end and at least one foot-end support.

9. The device as set forth in any one of claims 3 to 8, **characterised in that** it comprises adjusting means which cause the bed (3, 42, 50) to rotate about its lateral axis by purposefully setting the adjusting speed of at least one head-end and at least one foot-end support, whereby the position of the apparent axis of rotation may be placed in a desired position.

10. The device as set forth in any one of claims 3 to 9, **characterised in that** it comprises adjusting means which cause the bed (3, 42, 50) to rotate about its longitudinal axis by purposefully setting the adjusting speed of at least two supports, whereby the position of the apparent axis of rotation can be placed in a desired position.

## Revendications

1. Dispositif de positionnement d'un patient dans le cadre de la programmation d'un traitement avec saisie d'image et/ou exécution d'un traitement d'irradiation, qui présente une couchette pour patient (3, 42, 50) sur laquelle un patient est placé et un dispositif d'ajustement (6, 52) avec lequel la couchette pour patient (3, 42, 50) peut être tournée autour d'au moins deux axes et qui est disposée entre la couchette pour patient (3, 42, 50) et un dispositif d'appui de la couchette pour patient (3, 42, 50), en particulier une couche de programmation (8) ou une table de traitement (1), le dispositif d'ajustement (6, 52) étant constitué d'une plaque de base et d'une plaque de recouvrement (10, 12) qui sont reliées à déplacement mutuel par l'intermédiaire d'un support à quatre points, **caractérisé en ce que** le support de la plaque de recouvrement (12) sur la plaque de base (10) comprend une articulation (22, 23) qui dispose de deux degrés de liberté en translation et de deux degrés de liberté en rotation et qui empêche un déplacement latéral de la plaque de recouvrement (12) par rapport à la plaque de base (10).

2. Dispositif selon la revendication 1, **caractérisé en ce que** le dispositif d'ajustement (6, 52) permet une rotation indépendante de la couchette pour patient (3, 42, 50) par rapport à la couchette (3, 42, 50) et autour d'au moins un axe transversal et/ou d'au moins un axe longitudinal.

3. Dispositif selon les revendications 1 ou 2, **caractérisé en ce que** le dispositif d'ajustement (6, 52) présente au moins deux supports (16, 17) qui sont configurés comme supports pneumatiques, hydrauliques, piézoélectriques ou électromécaniques ajustables en hauteur qui peuvent être ajustés directement ou au moyen d'un système de leviers.

4. Dispositif selon les revendications 1 à 3, **caractérisé en ce que** le dispositif d'ajustement (6, 52) présente un support avant situé côté tête et un support arrière situé côté pieds, au moins l'un des supports étant configuré comme support à articulations sphériques ou comme support à cardans.

5. Dispositif selon la revendication 4, **caractérisé en ce que** le support avant ou le support arrière présentent un levier à genouillère doté d'articulations sphériques sur ses deux côtés.

6. Dispositif selon l'une des revendications 3 à 5, **caractérisé en ce qu'**il présente des moyens d'ajustement qui réalisent la rotation de la couchette (3, 42, 50) autour de son axe longitudinal par un ajustement en sens opposés de deux supports situés côté tête ou de deux supports situés côté pieds.

7. Dispositif selon l'une des revendications 3 à 6, **caractérisé en ce qu'**il présente des moyens d'ajustement qui réalisent la rotation de la couchette (3, 42, 50) autour de son axe transversal par un ajustement dans le même sens d'un ou deux supports situés côté tête ou situés côté pieds.

8. Dispositif selon l'une des revendications 3 à 7, **caractérisé en ce qu'**il présente des moyens d'ajustement qui réalisent la rotation de la couchette (3, 42, 50) autour de son axe transversal par ajustement simultané d'au moins un support situé côté tête et d'au moins un support situé côté pieds.

9. Dispositif selon l'une des revendications 3 à 8, **caractérisé en ce qu'**il présente des moyens d'ajustement qui réalisent la rotation de la couchette (3, 42, 50) autour de son axe transversal par un réglage contrôlé de la vitesse d'ajustement d'au moins un support situé côté tête et d'au moins un support situé côté pieds, grâce à quoi l'axe de rotation apparent peut être amené dans une position voulue.

10. Dispositif selon l'une des revendications 3 à 9, **caractérisé en ce qu'**il présente des moyens d'ajustement qui réalisent la rotation de la couchette (3, 42, 50) autour de son axe longitudinal par un réglage contrôlé de la vitesse d'ajustement d'au moins deux supports, grâce à quoi l'axe de rotation apparent peut être amené dans une position voulue.
